(19) Europäisches Patentamt
European Patent Office
Office européen des brevets

(11) Veröffentlichungsnummer: **0 492 078 A2**

(12) # EUROPÄISCHE PATENTANMELDUNG

(21) Anmeldenummer: **91118097.4**

(22) Anmeldetag: **24.10.91**

(51) Int. Cl.[5]: **C07C 69/743**, C07C 67/317, C07C 69/65, C07C 67/307

(30) Priorität: **22.12.90 DE 4041540**

(43) Veröffentlichungstag der Anmeldung: **01.07.92 Patentblatt 92/27**

(84) Benannte Vertragsstaaten: **AT BE CH DE FR GB IT LI NL SE**

(71) Anmelder: **HÜLS AKTIENGESELLSCHAFT**
**Patentabteilung / PB 15 - Postfach 13 20**
**W-4370 Marl 1(DE)**

(72) Erfinder: **Kaufhold, Manfred, Dr.**
**Jasminweg 20**
**W-4370 Marl(DE)**

(54) **Verfahren zur Herstellung von 2-Phenylcyclopropancarbonsäurebutylestern.**

(57) Die Erfindung betrifft ein Verfahren zur Herstellung von 2-Phenylcyclopropancarbonsäurebutylester durch Umsetzung von 5-Phenylbutyrolacton mit Alkohol und gasförmigem Chlorwasserstoff zum 4-Chlor-4-phenylbutansäurebutylester und dessen Cyclisierung mit einem Alkalialkoholat zum 2-Phenylcyclopropancarbonsäurebutylester, welches dadurch gekennzeichnet ist, daß man als Alkoholkomponente Butanole in beiden Stufen verwendet und zunächst in einer ersten Stufe 5-Phenylbutyrolacton und Butanol im Molverhältnis 1 : 1 bis 1 : 5 und Chlorwasserstoff in Abwesenheit eines Katalysators bei einer Temperatur von 0 bis 50 °C umsetzt, über eine Kolonne Butanol und Wasser abdestilliert und ohne Reinigung den rohen 4-Chlor-4-phenylbutansäurebutylester in die 2. Stufe einsetzt, bei der dieser Ester mit einer Lösung eines Natriumbutylats in dem entsprechenden Butanol bei 50 bis 150 °C und einem Molverhältnis von Alkoholat zur Chlorverbindung von 1 : 1 bis 3 : 1 cyclisiert.

EP 0 492 078 A2

Die Erfindung betrifft ein Verfahren zur Herstellung von 2-Phenylcyclopropancarbonsäurebutylestern ( = PCPB) der Formel (1) und dem entsprechenden Amid (2) durch Umsetzung von 5-Phenylbutyrolacton (3) mit Chlorwasserstoff und einem Butylalkohol zu einem 4-Chlor-4-phenylbutansäureester ( = CPBE) der Formel (4) und anschließende Cyclisierung mit einem Alkoholat zu (1) sowie Umsetzung mit Ammoniak zu Amid (2).

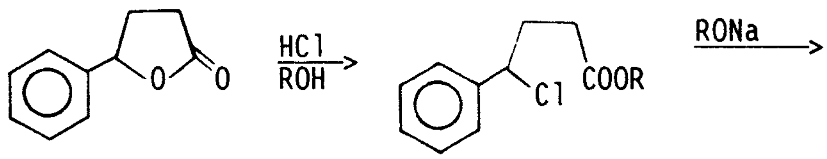


3 4

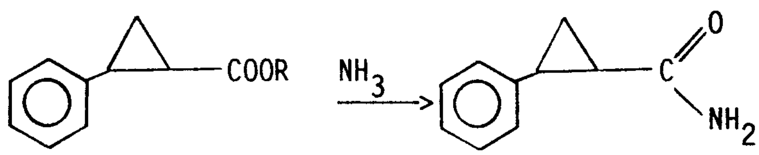


1 2

Synthesen von 4-Chlor-4-phenylbutansäureestern und 2-Phenylcyclopropancarbonsäureestern, die vom 5-Phenylbutyrolacton ausgehen, das durch Umsetzung von Acrylsäure mit Benzylalkohol leicht zugänglich ist, sind literaturbekannt.

So beschrieben U. und S. Julia und B. Bémont bereits 1960 zwei Synthesen von 4-Chlor-4-phenylbutansäureethylester und 2-Phenylcyclopropancarbonsäureethylester, ausgehend vom 5-Phenylbutyrolacton (Bull. Soc. Chim. France 1960, 304-12). Bei der Methode A wird eine Lösung von (3) in Ethanol gelöst, mit Chlorwasserstoff gesättigt, 48 Stunden lang stehengelassen, auf Eiswasser geschüttet, mit Ether extrahiert und destillativ aufgearbeitet. Die Ausbeute beträgt nur 66 %.

Bei der Methode B wird (3) zunächst mit Thionylchlorid umgesetzt, das Reaktionsprodukt zu einer Lösung von Chlorwasserstoff in Ethanol gegeben und destillativ aufgearbeitet. Die Ausbeute ist mit 97 % sehr hoch.

Die 2. Stufe, die Ringbildung, wird mit Natrium-tert.-amylat in Benzol innerhalb von 35 Stunden durchgeführt. Die Aufarbeitung erfolgt ähnlich wie in der 1. Stufe durch Behandeln mit Eiswasser, Extraktion mit Ether und Wäsche der Etherlösungen. Die destillative Aufarbeitung liefert das gewünschte Produkt in 77 %iger Ausbeute.

Diese Arbeitsweisen sind aufwendig und verursachen viel Abfallprodukte. Der Einsatz von beispielsweise Thionylchlorid ist kostenungünstig im Vergleich zum Einsatz von Chlorwasserstoff.

Die Verwendung von festem Natriumamylat in Benzol erfordert zusätzliche Kosten bei der Herstellung dieses Gemisches und ihrer Aufarbeitung nach der Reaktion.

Für analoge Ringschlußreaktionen wurde von R. Lantzsch (Synthesis 1982, 11, 955-956) ein Cyclisierungsverfahren vorgeschlagen, das mit der leicht handhabbaren wäßrigen Kalilauge arbeitet. Nachteilig bei dieser Arbeitsweise ist aber die zu hohe Verdünnung durch das erforderliche Lösungsmittel und die Verwendung von sehr großen Mengen an Phasentransfer-Katalysatoren - und zwar gewichtsmäßig mehr als ein Viertel des Gewichts des Einsatzprodukts, d. h. der zu 4 analogen Verbindung. Dadurch entstehen Abwasserprobleme und hohe Einsatzstoffkosten, welche die Vorteile der Kalilauge weit übertreffen.

Alle bekannten Verfahren benötigen teure Chemikalien, sind technisch sehr aufwendig und zeitraubend und führen zu Problemen der Abfallbeseitigung. Wünschenswert ist daher ein Verfahren, bei dem 5-Phenylbutyrolacton im Eintopfverfahren mittels Chlorwasserstoff und einem Alkohol in einen 4-Chlor-4-Phenylbutansäureester übergeführt und dieser drucklos in üblichen Reaktoren cyclisiert wird. Der entstandene 2-Phenylcyclopropancarbonsäureester kann beispielsweise katalytisch mit Ammoniak zum Amid umgesetzt werden.

Es besteht ein großes Interesse an einem derartigen Verfahren, nach dem man bei geringem technischen Aufwand und ohne den Einsatz von teuren Reagenzien aus 5-Phenylbutyrolacton 2-Phenylcyclopropancarbonsäurebutylester und aus diesem gegebenenfalls 2-Phenylcyclopropancarbonsäureamid herstellen kann, weil diese Produkte wichtige Rohstoffe für Pharma-Produkte sind.

Die sich hieraus ergebende Aufgabe wird erfindungsgemäß entsprechend den Angaben der Patentansprüche gelöst.

Gegenstand der Erfindung ist daher ein Verfahren zur Herstellung von 2-Phenylcyclopropancarbonsäurebutylester durch Umsetzung von 5-Phenylbutyrolacton mit Alkohol und gasförmigem Chlorwasserstoff zum 4-Chlor-4-phenylbutansäurebutylester und dessen Cyclisierung mit einem Alkalialkoholat zum 2-Phenylcyclopropancarbonsäurebutylester, welches dadurch gekennzeichnet ist, daß man als Alkoholkomponente Butanole in beiden Stufen verwendet und zunächst in einer ersten Stufe 5-Phenylbutyrolacton und Butanol im Molverhältnis 1 : 1 bis 1 : 5 und Chlorwasserstoff in Abwesenheit eines Katalysators bei einer Temperatur von 0 bis 50 °C umsetzt, über eine Kolonne Butanol und Wasser abdestilliert und ohne Reinigung den rohen 4-Chlor-4-phenylbutansäurebutylester in die 2. Stufe einsetzt, bei der dieser Ester mit einer Lösung des Natriumbutylats in dem entsprechenden Butanol bei 50 bis 150 °C und einem Molverhältnis von Alkoholat zur Chlorverbindung von 1 : 1 bis 3 : 1 cyclisiert.

Überraschenderweise ist die erste Reaktionsstufe, die Umsetzung des 5-Phenylbutyrolactons mit Chlorwasserstoff und Butanol, in sehr kurzer Zeit auf einfachste Weise durchführbar, wenn man bei Raumtemperatur arbeitet, die Reaktionswärme schnell abführt und anschließend das überschüssige Butanol bei niedrigen Temperaturen abdestilliert. Geeignete Butanole sind n-Butanol und Isobutanol. Die Umsetzung erfolgt bei 0 bis 50 °C, vorzugsweise bei 10 bis 20 °C, in Abwesenheit eines Katalysators.

Die Cyclisierung des 4-Chlor-4-phenylbutansäureisobutylesters in der zweiten Reaktionsstufe gelingt überraschend glatt bei Normaldruck und mit sehr guten Ausbeuten von über 95 %, wenn man bei relativ hohen Temperaturen die Chlorverbindung zu einer Lösung eines Natriumalkoholats in dem entsprechenden Alkohol, der 4 oder mehr C-Atome hat, gibt. Hierbei werden ungewöhnlich kurze Reaktionszeiten von z. B. 1 Stunde erreicht. Längere Reaktionszeiten verringern die Ausbeuten.

Dieser Befund, daß hohe Temperaturen und kurze Zeiten günstig sind, steht im Gegensatz zu den obengenannten Literaturstellen, die Cyclisierungen bei relativ tiefen Temperaturen durchführen und dann längere Reaktionszeiten benötigen. So wählte R. Lantzsch Reaktionstemperaturen von 35 bis 40 °C und benötigt für die Umsetzung 3 Stunden. Ausdrücklich weist er auf die Gefahr der Eliminierung von Chlorwasserstoff als Nebenreaktion hin, vor allem bei Einsatz von starken Basen und bei den von ihm untersuchten Verbindungen, bei denen es sich um tertiäre Chlorverbindungen handelt, die bekanntlich besonders instabil sind im Vergleich zu sekundären und primären Verbindungen.

Die vorliegende Chlorverbindung 4 ist besonders instabil, weil sie durch den Benzolring zusätzlich aktiviert ist und bei der Chlorwasserstoffeliminierung ein stabiles Styrolderivat entsteht:

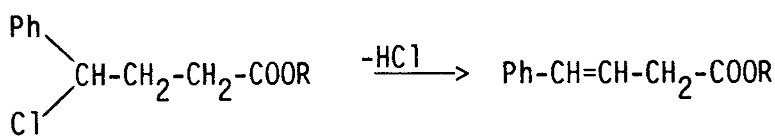


4 5

Darum ist der Befund besonders überraschend und neu.

Als Alkoholate bzw. Alkohole werden Butanole, insbesondere n-Butanol und Isobutanol eingesetzt. Alkohole mit 5 bis 20 C-Atomen bzw. die entsprechenden Alkoholate können zwar verwendet werden, bieten aber nicht die Vorteile gemäß der Erfindung.

Die Verwendung einer Natrium-n- oder -iso-butylatlösung im entsprechenden Butanol ist besonders kostengünstig, weil sie aus Natronlauge und n-Butanol oder mittels Natriummethylat einfach hergestellt werden kann, ohne daß ein luftempfindlicher Feststoff verarbeitet werden muß.

Ferner wurde überraschenderweise gefunden, daß der eingesetzte 4-Chlor-4-phenylbutansäurebutylester nicht durch Destillation gereinigt werden muß, sondern als Rohprodukt verwendet werden kann. Das ist wegen seiner geringen thermischen Stabilität ein großer Vorteil hinsichtlich Ausbeute und Produktionskosten.

Die praktische Durchführung der einzelnen Stufen erfolgt beispielsweise in folgender Weise:

Bei der Herstellung des 4-Chlor-4-phenylbutansäurebutylesters wird ohne Katalysator gearbeitet. 5-Phenylbutyrolacton und n- oder iso-Butanol wird im Molverhältnis 1 : 1 bis 1 : 5, vorzugsweise 1 : 2 bis 1 : 3, vorgelegt und bei Raumtemperatur Chlorwasserstoff eingeleitet, wobei Wärme entsteht, die abgeführt wird. Es wird so schnell Chlorwasserstoff eingeleitet, wie das Gas aufgenommen wird, bis zur Sättigung. Die Reaktionstemperatur wird auf 0 bis 50 °C, vorzugsweise auf 10 bis 20 °C, eingestellt. Wenn die

Gasaufnahme beendet ist, wird die Hauptmenge an Butanol abdestilliert und der Destillationsrückstand ohne Reinigung weiterverarbeitet.

Die Cyclisierung des 4-Chlor-4-phenylbutansäurebutylesters erfolgt bei 50 bis 150 °C, vorzugsweise bei 90 bis 110 °C, indem man beispielsweise eine Lösung von Natriumisobutylat in Isobutanol vorlegt, den 4-Chlor-4-phenylbutansäurebutylester zugibt und das Gemisch erhitzt.

Die Reaktionszeit muß so kurz wie möglich sein, weil sonst vermehrt Nebenprodukte gebildet werden.

Das Molverhältnis Alkoholat zum 4-Chlor-4-phenylbutansäurebutylester beträgt 1 : 1 bis 3 : 1, vorzugsweise 1,8 : 1 bis 1,1 : 1.

Den erhaltenen 2-Phenylcyclopropancarbonsäurebutylester setzt man beispielsweise in an sich bekannter Weise mit Ammoniak zum Amid um, das ein wichtiges Zwischenprodukt zur Herstellung von Pharma-Produkten ist. Zur Amidherstellung verwendet man ebenfalls eine Lösung von Natrium-n- oder -iso-butylat im entsprechenden Butanol als Katalysator, Butanol als Lösungsmittel und einem Ammoniakdruck von beispielsweise 12 bar bei Reaktionstemperaturen von z. B. 120 °C. Diese Ausführungsform ist vorteilhaft, weil nach der Reaktion beim Abkühlen das Amid kristallin ausfällt und auf einfachste Weise durch Filtration in reiner Form gewonnen wird. Die Mutterlauge ist wieder einsetzbar.

Die folgenden Beispiele sollen das erfindungsgemäße Verfahren näher erläutern.

Beispiel 1a: **4-Chlor-4-phenylbutansäureisobutylester ( = CPBE) (1. Stufe)**

Man benutzt eine Glasapparatur, die aus einem Vierhalskolben mit Gaseinleitungsrohr, Rührer, Thermometer, Tropftrichter und Rückflußkühler besteht.

Man setzt ein: 711 g (3,99 Mol) 5-Phenylbutyrolacton (91,1 %ig)
741 g (10 Mol) Isobutanol

Die Einsatzprodukte werden vorgelegt und unter Kühlen mit einem Eisbad so schnell Chlorwasserstoff eingeleitet, wie das Gas von der Lösung aufgenommen wird. Die Temperaturen schwanken zwischen 10 bis 20 °C. Nach 3 Stunden ist die Aufnahme beendet. Der Gehalt an Zielprodukt liegt nach GC-Analyse, lösungsmittelfrei gerechnet, bei 82,8 %.

Im Vakuum bei 130 bis 50 mbar wird überschüssiger Chlorwasserstoff und die Hauptmenge an Isobutanol, 536,5 g, abdestilliert. Der Destillationsrückstand, 1 079 g, enthält neben 1,3 % Isobutanol 88,2 % CPBE, das entspricht einer Ausbeute von 952 g CPBE = 93,6 % der Theorie, bezogen auf Einsatz. Ohne Reinigung wird dieses Produkt weiterverarbeitet.

Beispiel 1b: **2-Phenylcyclopropancarbonsäureisobutylester ( = PCPB) (2. Stufe)**

Man benutzt eine Glasapparatur, die aus einem Dreihalskolben mit Rührer, Thermometer, Tropftrichter und 0,5 m langer mit Glasraschigringen gefüllter Glaskolonne mit Destillationsaufsatz besteht.

Man setzt ein: 143 g (0,79 Mol) Natriummethylatlösung (30 %ig)
196 g (2,6 Mol) Isobutanol
200,5 g (0,69 Mol) CPBE 88,2 % aus Stufe 1

Isobutanol und die Natriummethylatlösung werden vorgelegt und durch Abdestillieren des Methanols und wenig Isobutanol, 204 g, einer Lösung des Natriumisobutylats in Isobutanol hergestellt. Dann wird bei 100 °C innerhalb von 0,5 Stunden CPBE zugetropft und noch 1 Stunde bei dieser Temperatur gerührt. Nach dem Abkühlen wird mit 175 g Wasser, das 7,5 % Phosphorsäure enthält, gewaschen und mit 130 g einer wäßrigen Kochsalzlösung nachgewaschen. Die GC-Analyse der organischen Phase, 251,5 g, wasserhaltig = 236 g, wasserfrei gerechnet, zeigte folgende Zusammensetzung:

| | | |
|---|---|---|
| Isobutanol | 33,1 % | |
| unbekannte Komponente | 1,4 % | |
| cis-PCPB | 2,7 % | } 61,7 % |
| trans-PCPB | 59,0 % | |
| | 96,2 % | |

Hieraus errechnet sich eine Ausbeute an gebildetem cis- und trans-PCPB von 146 g = 96,7 % der Theorie,

bezogen auf Einsatz. Die destillative Aufarbeitung lieferte im Siedebereich von 157 bis 158 °C bei 30 mbar das PCPB mit einem trans-Gehalt von über 90 %.

Beispiele 2 bis 5: **(2. Stufe)**

Man benutzt die in 1b beschriebene Apparatur und setzt die hier angegebenen Einsatzstoffmengen ein. Die Reaktionszeit wird auf 1 Stunde begrenzt und Reaktionstemperaturen von 50, 70, 80, 100 und 120 °C gewählt. Hierbei ergeben sich folgende Zusammenhänge zwischen Reaktionstemperatur (RT) und Ausbeute:

| RT in °C | Ausbeute in % |
|---|---|
| 50 | 71 |
| 70 | 75 |
| 80 | 81 |
| 100 | 97 |
| 120 | 90 |

## Patentansprüche

1. Verfahren zur Herstellung von 2-Phenylcyclopropancarbonsäurebutylester durch Umsetzung von 5-Phenylbutyrolacton mit Alkohol und gasförmigem Chlorwasserstoff zum 4-Chlor-4-phenylbutansäurebutylester und dessen Cyclisierung mit einem Alkalialkoholat zum 2-Phenylcyclopropancarbonsäurebutylester,
   dadurch gekennzeichnet,
   daß man als Alkoholkomponente Butanole in beiden Stufen verwendet und zunächst in einer ersten Stufe 5-Phenylbutyrolacton und Butanol im Molverhältnis 1 : 1 bis 1 : 5 und Chlorwasserstoff in Abwesenheit eines Katalysators bei einer Temperatur von 0 bis 50 °C umsetzt, über eine Kolonne Butanol und Wasser abdestilliert und ohne Reinigung den rohen 4-Chlor-4-phenylbutansäurebutylester in die 2. Stufe einsetzt, bei der dieser Ester mit einer Lösung eines Natriumbutylats in dem entsprechenden Butanol bei 50 bis 150 °C und einem Molverhältnis von Alkoholat zur Chlorverbindung von 1 : 1 bis 3 : 1 cyclisiert.

2. Verfahren nach Anspruch 1,
   dadurch gekennzeichnet,
   daß man in der ersten Stufe 5-Phenylbutyrolacton und Butanol im Molverhältnis 1 : 2 bis 1 : 3 einsetzt.

3. Verfahren nach den Ansprüchen 1 und 2,
   dadurch gekennzeichnet,
   daß man die Umsetzung der ersten Stufe bei 10 bis 20 °C durchführt.

4. Verfahren nach Anspruch 1,
   dadurch gekennzeichnet,
   daß man in der zweiten Stufe bei einem Molverhältnis Alkoholat zu 4-Chlor-4-phenylbutansäurebutylester von 1,8 : 1 bis 1,1 : 1 cyclisiert.

5. Verfahren nach den Ansprüchen 1 und 4,
   dadurch gekennzeichnet,
   daß man in der zweiten Stufe bei einer Temperatur von 90 bis 110 °C cyclisiert.

6. Verwendung des 2-Phenylcyclopropancarbonsäurebutylesters nach Anspruch 1 zur Herstelung des entsprechenden Amids.